# EUROPÄISCHE PATENTANMELDUNG

(11) **EP 1 790 299 A2**
(43) Veröffentlichungstag der Anmeldung: **30.05.2007**
(21) Anmeldenummer: 06024512.3
(22) Anmeldetag: 27.11.2006
(51) Int. Cl.: A61B 17/32, A61M 1/00

(54) **Chirurgische Einrichtung zum Trennen einer biologischen Struktur mit Hilfe eines Flüssigkeitsstrahls**

(30) Priorität: 29.11.2005 DE 202005018601 U
(71) Anmelder: Human Med AG, 19061 Schwerin (DE)
(72) Erfinder: Winkler, Konrad-Wenzel, 19417 Warin (DE)
(74) Vertreter: Jaap, Reinhard

(57) **Zusammenfassung**

Um die Funktionspalette einer gattungsgemäßen chirurgischen Einrichtung zu erweitern, wird vorgeschlagen, dass zur chirurgischen Einrichtung ein HF-Generator (16) gehört, der über eine elektrische Leitung (17,17') mit dem Absaugrohr (21, 21') des Operationshandstück (2) und mit einer zweiten Elektrode verbunden ist, wobei das Operationshandstück (2) aus einem elektrisch leitenden Material besteht und am distalen Ende des Absaugrohres (21, 21') eine elektrische Kontaktfläche (27) ausbildet und die elektrische Kontaktfläche (27) und die zweite Elektrode zum Einspannen von Gewebeteilen ausgelegt ist.

## Beschreibung

Die Erfindung bezieht sich auf eine chirurgische Einrichtung nach dem Oberbegriff des Anspruchs 1.
Derartige Einrichtungen werden in chirurgischen Kliniken, insbesondere für offene Eingriffe eingesetzt.

Es hat sich in der Medizin inzwischen durchgesetzt, biologische Strukturen in der minimal invasiven als auch in der offenen Chirurgie mit der Kraft eines Wasserstrahls zu trennen. Dazu wird jeweils eine Wasserstrahltrenneinrichtung eingesetzt, zu der im Wesentlichen ein Druckstromerzeuger zum Aufbau eines trennenden Flüssigkeitsstrahles, eine Vakuumpumpe zum Absaugen von Flüssigkeiten und von gelösten Gewebezellen und ein Operationshandstück gehört. Das Opcrationshandstück besteht aus einem äußeren Absaugrohr mit ein oder mehreren Absaugöffnungen und einer inneren und koaxial zum Absaugrohr angeordneten Einspritzkanüle mit einer am distalen Ende befindlichen Einspritzdüse. Die Ausgestaltung des Operationshandstücks ist in besonderer Weise auf die unterschiedlichsten Anwendungen abgestimmt.

So ist beispielsweise aus der DE 100 33 278 A1 ein Operationshandstück für einen minimal invasiven Eingriff zum Entfernen von Fettgewebe bekannt, bei dem das Absaugrohr an seinem distalen Ende verschlossen und nur mit einer Führungsbohrung zur Hindurchführung der Einspritzkanüle ausgeführt ist. Die für die Absaugung erforderlichen Absaugöffnungen sind in mehrfacher Anzahl radial ausgerichtet und am Umfang des Absaugrohres verteilt angeordnet. Mit diesem Operationshandstück wird das Fettgewebe im vorderen Bereich der Einspritzdüse abgetrennt und im hinteren Bereich der Absaugöffnungen abgesaugt.

Aus der Produktpalette der Firma Human Med AG, 19061 Schwerin ist unter dem Namen Helix-Hydro-Jet eine Wasserstrahltrenneinrichtung bekannt, die ausweislich eines Firmenprospektes ein Operationshandstück sowohl für die minimal invasive als auch für die offene Chirurgie verwendet. Dieses Operationshandstück besitzt ein äußeres Absaugrohr, das an seinem distalen Ende offen ist und eine innere und koaxial zum Absaugrohr ausgerichtete Einspritzkanüle. Damit ergibt sich zwischen den Absaugrohr und der Einspritzkanüle ein im Querschnitt ringförmiger Absaugkanal mit einer ebenfalls im Querschnitt ringförmigen Absaugöffnung am distalen Ende des Absaugrohres. Innerhalb des Absaugrohres befindet sich ein Abstandshalter, der die Einspritzkanüle koaxial gegenüber dem Absaugrohr ausrichtet und fixiert.

Bei der Anwendung dieser Operationshandstücke werden zur anschließenden Koagulation der aufgetrennten Gewebeteile und Tuben spezielle Instrumente verwendet, was bedeutet, dass der Operateur das chirurgische Instrument gegen ein Koagulationsinstrument austauschen muss. In der Regel kommen hochfrequente Elektroinstrumente zum Einsatz, wobei diese HF-Instrumente monopolar als auch bipolar ausgeführt sein können.
Im Prospekt der Firma Sutter Medizintechnik GmbH, 79108 Freiburg aus dem Jahre 2004 werden eine Vielzahl von bipolaren Pinzetten vorgestellt. Jede Pinzette besteht aus zwei elektrisch leitenden Greifschenkeln, die am proximalen Ende zu einer elektrischen Steckkupplung zusammengeführt und die am distalen Ende mit ihren Schenkelspitzen voneinander beabstandet sind. Mit Ausnahme des Kupplungsbereiches und der Schenkelspitzen sind beide Greifschenkel elektrisch isoliert, wobei an beiden Greifschenkeln im kupplungsnahen Bereich Hand- und Betätigungsgriffe ausgebildet sind. Mit den Schenkelspitzen der bipolaren Pinzette wird das offene Gewebe zusammengedrückt und mit einem hochfrequenten Strom versorgt, wodurch eine elektrische Verbindung zwischen den beiden Schenkelspitzen und über das eingeklemmte Gewebe kurz geschlossen wird und das Gewebe unter dem Einfluss der entstehenden Hitze koaguliert.
Diese Pinzette gibt es gemäß der Abbildung auf der Seite 2.6 des Firmenprospektes auch als bipolare Spülpinzette. Dazu ist ein Greifschenkel hohlförmig und mit einem proximalen Einlassanschluss zum Einleiten einer Spülflüssigkeit und einer distalen Austrittsöffnung für die Spülflüssigkeit ausgeführt. Die distale Austrittsöffnung ist radial ausgerichtet und befindet sich auf der Innenseite der entsprechenden Schenkelspitze. Damit wird die Spülflüssigkeit während der Koagulation und unter einem sehr geringen Verteilerdruck auf das OP-Feldes gespült, um das OP-Feld sauber zu halten und um ein Austrocknen des Gewebes zu verhindern.
Auf der Seite 24 des genannten Firmenprospektes ist weiterhin eine bipolare Saugpinzette abgebildet. Auch bei dieser Saugpinzette ist ein Schenkel hohlförmig ausgebildet und mit einer distalen Saugöffnung an der Schenkelspitze und einem proximalen Auslassanschluss ausgestattet. Dabei ist die distale Saugöffnung axial ausgerichtet. Mit dieser Ausführung werden vor und während der Koagulation Blut, Sekrete und Rauch abgesaugt, um immer ein freies Sichtfeld während des Eingriffs zu gewährleisten.

Für einen chirurgischen Eingriff werden also zunächst das chirurgischen Instrument zum Trennen der biologischen Struktur, dann die bipolare Pinzette zum Spülen und Koagulieren und dann die bipolare Pinzette zum Koagulieren und Absaugen benötigt. Damit ist die erforderliche Gerätetechnik aufwendig und teuer. Die Handhabung der vielen Instrumente erfordert auch viel Zeit, die die Operationszeit unnötig verlängert und den Patienten zusätzlich belastet. Obendrein leidet die Qualität der Operation unter der Instrumentenvielfalt, weil die vielen Handgriffe zum Wechseln der einzelnen Instrumente die Konzentration des Operateurs schwächen.

Die Aufgabe der Erfindung besteht also darin, die Funktionspalette einer gattungsgemäßen chirurgischen Einrichtung zu erweitern.
Diese Aufgabe wird durch die kennzeichnenden Merkmale des Anspruches 1 gelöst. Zweckdienliche Ausgestaltungen ergeben sich aus den Unteransprüchen 2 bis 7.
Die neue chirurgische Einrichtung beseitigt die genannten Nachteile des Standes der Technik. Dabei ist von besonderem Vorteil, dass dem Operateur für seinen Eingriff einfachste Bedingungen gestellt werden, damit er sich ganz auf die Operation konzentrieren kann.
Die neue chirurgische Einrichtung vereint das bisher bekannte Trennen von Gewebe und Absaugen der abgetrennten Gewebeteile mit Hilfe eines Wasserstrahles und das anschließende Koagulieren mit dem Spülen der Schnittstelle im Gewebe und dem Absaugen der Spülflüssigkeit. Dazu waren vorher mit der Wasserversorgungseinrichtung und dem HF-Generator zwei Einrichtungen und mit dem chirurgischen Instrument des Wasserstrahlens, der Spülpinzette und der Saupinzette des Koagulierens drei verschiedene Operationshandstücke erforderlich. Der Geräteaufwand wird also erheblich eingespart.
Dabei ist weiter von Vorteil, dass diese Einrichtung sowohl für den monopolaren Betrieb als auch für den bipolaren Betrieb ausgelegt ist. Trotzt der Multifunktionalität bleiben die die Qualitäten der Einzelfunktionen erhalten.
Es ist bei der bipolaren Ausführung von Vorteil, wenn das Absaugrohr und die Einspritzkanüle koaxial ausgeführt und in einem der beiden Greifschenkel der Pinzette untergebracht werden. Das hat den Vorteil, dass die Pinzette zum Trennen und Absaugen des Gewebes nicht ständig zusammen gedrückt werden muss. Andererseits ist es von Vorteil, wenn beide Greifschenkel der Pinzette hohlförmig ausgeführt sind und das Absaugrohr dem einen Greifschenkel und die Einspritzkanüle dem anderen Greifschenkel zugeordnet wird. Dadurch können die Außendurchmesser der beiden Greifschenkel minimiert werden.
Von besonderem Vorteil ist aber, wenn die beiden Greifschenkel der Pinzette sich scherenartig kreuzen und damit die Betätigungsrichtung der beiden Greifschenkel umgekehrt wird. Das erspart dem Operateur, die Pinzette während des relativ langen Trenn- und Absaugvorganges ständig zusammen zu drücken.

Die Erfindung soll anhand von mehreren Ausführungsbeispielen näher erläutert werden. Dazu zeigen:
- Fig. 1:: eine chirurgische Einrichtung mit einem Operationshandstück in einer monopolaren Ausführungsform,
- Fig. 2:: eine Seitenansicht des Operationshandstücks in einer bipolaren Ausführungsform,
- Fig. 3:: eine Draufsicht des Operationshandstücks nach der Fig. 2,
- Fig. 4:: eine Seitenansicht eines Operationsbandstücks in einer anderen bipolaren Ausführungsform und
- Fig. 5:: eine Draufsicht des Operationshandstücks nach der Fig. 4.

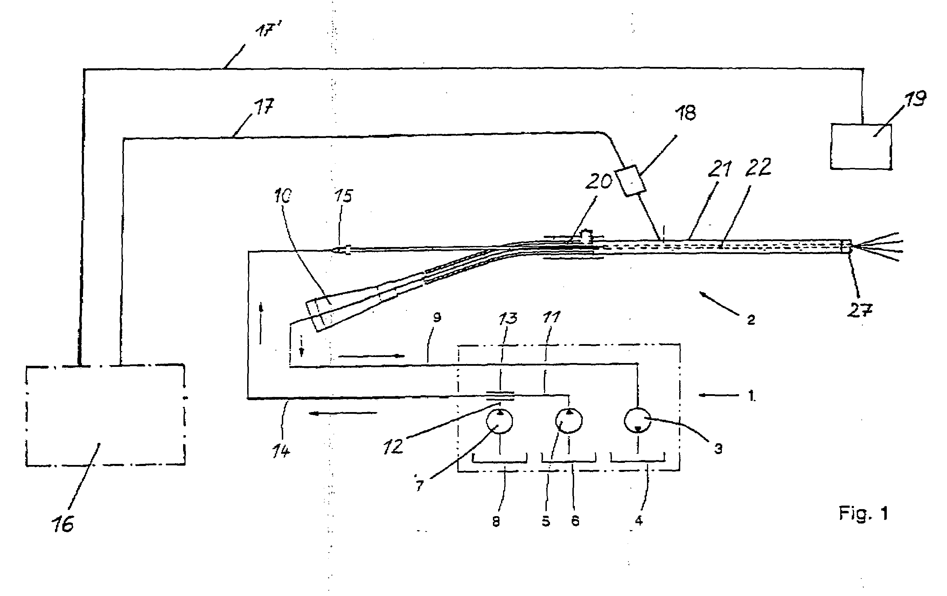

Nach der Fig. 1 besteht die chirurgische Einrichtung aus einer hydraulischen Versorgungseinrichtung 1 und einem Operationshandstück 2. Zur Versorgungseinriebtung 1 gehören eine Vakuumpumpe 3 mit einem Aufnahmebehälter 4, eine hydraulische Versorgungspumpe 5 mit einem Versorgungsbehälter 6 für eine sterile und strahlfähige Arbeitsflüssigkeit und eine hydraulische Versorgungspumpe 7 mit einem Versorgungsbehälter 8 für ein Anästhetikum oder einer anderen Arbeitsflüssigkeit.
Die Vakuumpumpe 3 ist über eine Unterdruckleitung 9 mit einer Kupplung 10 des Operationshandstücks 2 verbunden. Dagegen besitzen die beiden hydraulischen Versorgungspumpen 5 und 7 jeweils eine Druckleitung 11 und 12, die beide in ein schaltbares Wegeventil 13 münden. Dieses Wegeventil 13 ist für den Einsatz der beiden Versorgungspumpen 5 und /oder 7 vorgesehen und ist verbraucherseitig über eine Druckversorgungsleitung 14 und einer Kupplung 15 mit dem Operationshandstück 2 verbunden.
Zur chirurgischen Einrichtung gehört weiterhin ein elektrischer HF-Generator 16, der monopolar und bipolar betrieben werden kann.

Die Fig. 1 zeigt einen monopolaren HF-Stromkreis, bei dem der HF-Generator 16 einerseits über eine aktive elektrische Leitung 17 mit einer elektrischen Kupplung 18 des Operationshandstücks 2 verbunden ist und andererseits über eine neutrale elektrische Leitung 17' zu einer Kontaktelektrode 19 führt. Diese Kontaktelektrode 19 ist relativ großflächig ausgelegt und mit einem Körperteil des Patienten in Verbindung gebracht. Das in der Fig. 1 dargestellte, monopolare Operationshandstück 2 besteht aus einem Griffelement 20 und einem daran befestigten Absaugrohr 21. In diesem Absaugrohr 21 befindet sich eine Einspritzkanüle 22, die koaxial zum Absaugrohr 21 ausgerichtet ist. Dabei ist der Innendurchmesser des Absaugrohres 21 und der Außendurchmesser der Einspritzkanüle 22 so aufeinander abgestimmt, dass sich ein ringförmiger Absaugkanal mit einem für den Transport von Gewebeteilen ausreichenden Querschnitt ergibt. Das Absaugrohr 21 ist an seinem distalen Ende offen, sodass sich an dieser Stelle eine ringförmige Absaugöffnung ausbildet. Das Absaugrohr 21 besteht aus einem elektrisch leitenden Material und ist über die elektrische Kupplung 18 und der aktiven Leitung 17 mit dem HF-Generator 16 verbunden, Das Absaugrohr 21 ist über einen Großteil seiner Länge isoliert und bildet am distalen Ende des Absaugrohres 21 eine aktive Elektrode aus.
Mit dieser chirurgischen Einrichtung und dem monopolar ausgeführten Operationshandstück 2 wird eine biologische Struktur durch den unter Druck aus der Einspritzdüse austretenden Flüssigkeitsstrahl getrennt und die abgetrennten Gewebeteile zusammen mit der eingespritzten Flüssigkeit über das Absaugrohr 21 abgesaugt. Zur Koagulation der Schnittflächen an der biologischen Struktur wird der HF-Generator 16 aktiviert, wodurch ein elektrischer Strom zwischen der aktiven, am Patienten angelegten Kontaktelektrode 19 und der neutralen Elektrode am Absaugrohr 21 und durch den menschlichen Körper fließt. Auf Grund der Größenverhältnisse zwischen der aktiven Kontaktelektrode 19 und der neutralen Elektrode am Absaugrohr 21 entsteht im Bereich der neutralen Elektrode die größte Erwärmung, sodass das Gewebe in diesem Bereich erhitzt und zum Schmelzen gebracht wird. Die Schnittflächen sind somit verschlossen.

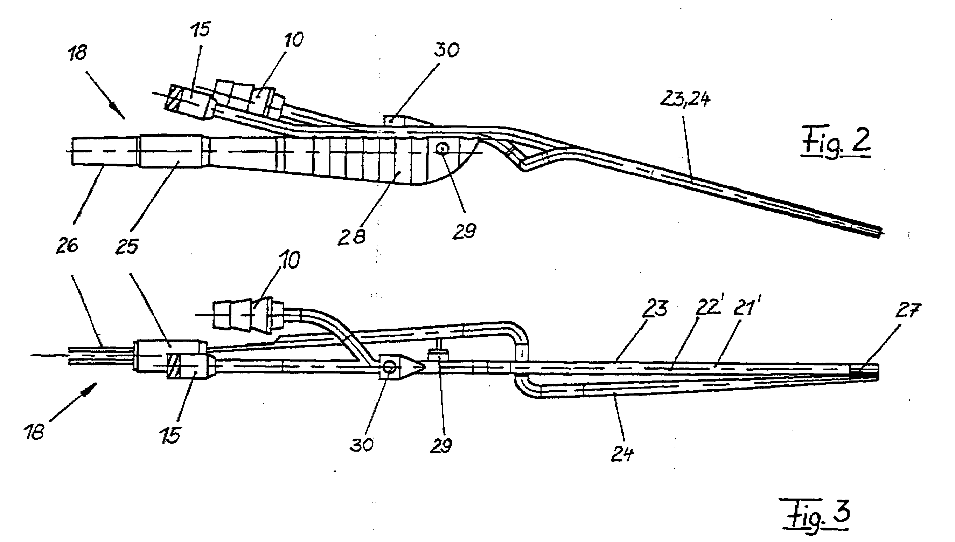

Ein bipolarer HF-Stromkreis ist nicht figürlich dargestellt. Er unterscheidet sich vom in der Fig. 1 gezeigten monopolaren Stromkreis lediglich dadurch, dass keine Kontaktelektrode 19 vorhanden ist und die aktive elektrische Leitung 17 und die neutrale elektrische Leitung 17' zusammen zur elektrischen Kupplung 18 des Operationshandstückes 2 geführt werden.
Das dementsprechende bipolare Operationshandstück 2 besitzt nach den Fig. 2 bis 5 die Form einer Pinzette mit zwei federnd gelagerten Greifschenkel 23, 24, die beide am proximalen Ende des Operationshandstückes 2 von einem Lagerstück 25 zusammengehalten und entgegen der federnden Vorspannung bewegt werden. Dieses Lagerstück 25 besitzt elektrische Kontaktelemente 26 zur leitfähigen Verbindung zwischen der zum HF-Generator 16 führenden zweipoligen elektrischen Leitung 17, 17' und jedem der beiden Greifschenkel 23, 24, wobei ein Greifschenkel 23, 24 als neutrale Elektrode und der andere Greifschenkel 23,24 als aktive Elektrode ausgeführt ist und die beiden am distalen Ende angeordneten Spitzen der beiden Greifschenkel 23, 24 jeweils mit elektrisch leitfähigen Kontaktflächen 27 für den gegenseitigen Kontakt ausgestattet sind. Die übrigen Bereiche des Operationshandstücks 2 zwischen dem Lagerstück 25 und den Spitzen der beiden Greifschenkel 23, 24 sind elektrisch isoliert.

Beide Greifschenkel 23, 24 besitzen aus ergonomischen Gründen im nahen Bereich des Lagerstücks 25 jeweils ein Griffelement 28 und beide Greifschenkel 23, 24 sind ebenfalls aus ergonomischen Gründen unmittelbar hinter den beiden Griffelementen 28 in der Höhe abgewinkelt. An den Innenseiten der beiden Greifschenkel 23, 24 befinden sich zwei gegenüberliegende Zentrierelemente 29, die mit geringer werdender Griffweite der beiden Greifschenkel 23, 24 ineinander greifen und beide Greifschenkel 23,24 im zusammengedrückten Zustand zueinander ausrichten und fixieren.

In einer ersten Ausführungsform, des bipolaren Operationshandstückes 2 besteht einer der beiden Greifschenkel 23, 24 gemäß der Fig. 2 und 3 aus einem äußeren Absaugrohr 21' und einer koaxial im Inneren des Absaugrohres 21' angeordneten Einspritzkanüle 22'. Dabei sind der Innendurchmesser des Absaugrohres 21' und der Außendurchmesser der Einspritzkanüle 22' so aufeinander abgestimmt, dass sich ein für den Transport von Flüssigkeit und Gewebeteile ausreichender ringförmiger Querschnitt ergibt. Am distalen Ende ist das Absaugrohr 21' offen ausgeführt, sodass sich an der Stirnseite des Greifschenkels 23 24 eine ringförmige Absaugöffnung ausbildet. Nicht dargestellt ist ein Halteelement, das im Inneren des Absaugrohres 21' angeordnet ist und das die Einspritzkanüle 22' zum Absaugrohr 21' auf Abstand hält. Hinter dem Griffelement 28 wird die innere Einspritzkanüle 22' aus dem Absaugrohr 21' herausgeführt und über die Kupplung 15 zusammen mit der Druckversorgungsleitung 14 fixiert, während das Absaugrohr 21' mit der zur Unterdruckleitung 9 führenden Kupplung 10 verbunden ist. Im Bereich des Griffelementes 28 besitzt das Absaugrohr 21' des einen Greifschenkels 23, 24 eine Bypasssaugöffnung 30, die mit einem Finger des Operateurs für den Ansaugvorgang verschlossen und zur Beendigung des Absaugvorganges wieder geöffnet werden kann.

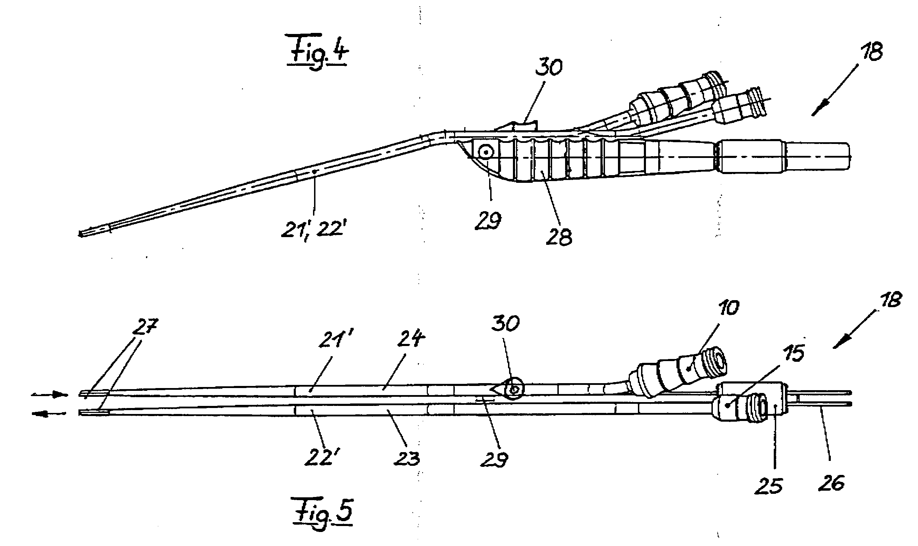

In einer zweiten Ausführungsform des bipolaren Operationshandstückes 2 gemäß der Fig. 4 und 5 sind beide Greifschenkel 23, 24 hohlförmig ausgeführt, wobei ein Greifschenkel 23, 24 als Absaugrohr 21' mit einem größeren Innendurchmesser und der andere Greifschenkel 23, 24 als eine Einspritzkanüle 22' mit einem etwas kleineren Innendurchmesser ausgebildet sind. Die Einspritzkanüle 22' besitzt an ihrem distalen Ende eine axiale Austrittsdüse und ist am anderen Ende über die Kupplung 15 mit der Druckversorgungsleitung 14 verbunden. Das Absaugrohr 21' besitzt an seinem distalen Ende eine axiale Absaugöffnung und eine gegenüberliegende Verbindung zur Kupplung 10, die zur Unterdruckleitung 9 führt. Das Absaugrohr 21' besitzt ebenfalls die Bypasssaugöffnung 30.

Mit dieser chirurgischen Einrichtung und den beiden Ausführungsformen eines bipolar ausgeführten Operationshandstück 2 als koaxiale oder parallele Saug- und Druckleitung wird eine biologische Struktur durch den unter Druck aus der Einspritzdüse austretenden Flüssigkeitsstrahl getrennt und die abgetrennten Gewebeteile zusammen mit der eingespritzten Flüssigkeit über das Absaugrohr 21' abgesaugt. Dabei werden die beiden Greifschenkel 23, 24 ständig von der Handkraft des Operateurs zusammengepresst, sodass die Spitzen beider Greifschenkel 23, 24 zur Anlage kommen.
Zur Koagulation der Schnittflächen an der biologischen Struktur wird die Pinzette geöffnet, sodass die beiden Greifschenkel 23, 24 auseinander gehen. Danach wird das zu koagulierende Gewebe mit der geöffneten Pinzette erfasst und zusammengepresst.
Sogleich wird der HF-Generator 16 aktiviert, wodurch ein elektrischer Strom zwischen den beiden. Kontaktflächen 27 und durch das eingeklemmte Gewebe fließt. Dabei erhitzt sich das Gewebe und verschmilzt.

Die Fig. 3 zeigt eine besondere Art der Pinzettenform, bei der sich beide Greifschenkel 23, 24 auf der Länge zwischen den Griffelementen 28 und den Spitzen der beiden Greifschenkel 23, 24 scherenartig kreuzen. Dadurch liegen die beiden Kontaktflächen 27 der Spitzen der beiden Greifschenkel 23, 24 im unbelasteten Zustand der Pinzette aneinander an. Im Gegensatz zur Eingangs beschriebenen Pinzettenform werden die beiden Greifschenkel 23, 24 durch die Handkraft des Operateurs nicht zusammen, sondern auseinander gedrückt und geöffnet.
Während der Trennung und der Absaugung bleiben die beiden Greifschenkel 23, 24 allein durch die federnde Vorspannung zusammen. Danach wird zur Koagulation der Schnittflächen die Pinzette durch die Handkraft des Operateurs kurz zusammengedrückt und damit geöffnet. Das Gewebe wird mit der geöffneten Pinzette ergriffen und dann die Handkraft wieder weggenommen. Durch die Kraft der federnden Vorspannung schließt sich die Pinzette wieder und presst das Gewebe zusammen. Danach wird der HF-Generator 16 aktiviert, sodass das Gewebe verschmilzt.

### Liste der Bezugszeichen

- 1: Versorgungseinrichtung
- 2: Operationshandstück
- 3: Vakuumpumpe
- 4: Aufnahmebehälter
- 5: hydraulische Versorgungspumpe
- 6: Versorgungsbehälter
- 7: hydraulische Versorgungspumpe
- 8: Versorgungsbehälter
- 9: Unterdruckleitung
- 10: Kupplung
- 11: Druckleitung
- 12: Druckleitung
- 13: schaltbares Wegeventil
- 14: Druckversorgungsleitung
- 15: Kupplung
- 16: elektrischer HF-Generator
- 17: elektrische Leitung
- 18: elektrische Kupplung
- 19: elektrische Kontaktelektrode
- 20: Griffelement
- 21: Absaugrohr
- 22: Einspritzkanüle
- 23: Greifschenkel
- 24: Greifschenkel
- 25: Lagerstück
- 26: elektrisches Kontaktelement
- 27: elektrische Kontaktfläche
- 28: Griffelement
- 29: Zentrierelement
- 30: Bypasssaugöffnung

## Patentansprüche

1. Chirurgische Einrichtung zum Trennen einer biologischen Struktur mit Hilfe eines Flüssigkeitsstrahls, bestehend aus einer Versorgungspumpe (5, 7) zur Erzeugung des trennenden Flüssigkeitsstrahls, einer Vakuumpumpe (3) zur Entsorgung des abgetrennten Gewebes und der eingebrachten Flüssigkeit und aus einem Operationshandstück (2) mit einer Einspritzkanüle (22) und einem Absaugrohr (21), wobei die Einspritzkanüle (22) eine Einspritzdüse und das Absaugrohr (21) eine Absaugöffnung besitzen, die am distalen Ende des Operationshandstückes (2) angeordnet und axial ausgerichtet sind,
**dadurch gekennzeichnet, dass** zur chirurgischen Einrichtung ein HF-Generator (16) gehört, der über eine elektrische Leitung (17, 17') mit dem Absaugrohr (21, 21') des Operationshandstück (2) und mit einer zweiten Elektrode verbunden ist, wobei das Operationsbandstück (2) aus einem elektrisch leitenden Material besteht und am distalen Ende des Absaugrohres (21, 21') eine elektrische Kontaktfläche (27) ausbildet und die elektrische Kontaktfläche (27) und die zweite Elektrode zum Einspannen von Gewebeteilen ausgelegt ist.

2. Chirurgische Einrichtung nach Anspruch 1, wobei die Einspritzkanüle (22) im Inneren des Absaugrohres (21) angeordnet und koaxial zum Absaugrohr (21) ausgerichtet ist,
**dadurch gekennzeichnet, dass** das Operationshandstück (2) für eine monopolare Koagulation ausgelegt ist und dazu die zweite Elektrode als eine externe, an ein Körperteil des Patienten anlegbare Kontaktelektrode (19) ausgebildet ist.

3. Chirurgische Einrichtung nach Anspruch 1,
**dadurch gekennzeichnet, dass** das Operationshandstück (2) für eine bipolare Koagulation ausgelegt ist und dazu die Form einer Pinzette mit zwei federnd gelagerten Greifschenkel (23, 24) besitzt, wobei jeder Greifschenkel (23, 24) mit einem der beiden elektrischen Leitungen (17, 17') des HF-Generators (16) verbunden ist und beide Greifschenkel (23, 24) an den Innenseiten ihrer Spitzen mit elektrischen Kontaktflächen (27) ausgestattet sind.

4. Chirurgische Einrichtung nach Anspruch 3,
**dadurch gekennzeichnet, dass** einer der beiden Greifschenkel (23, 24) hohlförmig ausgebildet ist und aus einem äußeren Absaugrohr (21') und einer inneren Einspritzkanüle (22') besteht, die beide koaxial zueinander ausgerichtet sind.

5. Chirurgische Einrichtung nach Anspruch 3,
**dadurch gekennzeichnet, dass** beide Greifschenkel (23, 24) hohlförmig ausgebildet sind und ein Greifschenkel (23, 24) aus einer Einspritzkanüle (22') und der andere Greifschenkel (23, 24) aus einem Absaugrohr (21') besteht.

6. Chirurgische Einrichtung nach dem Anspruch 4 oder 5,
**dadurch gekennzeichnet, dass** sich beide Greifschenkel (23, 24) scherenartig kreuzen und so unter Spannung stehen, dass die beiden Kontaktflächen (27) der Spitzen der beiden Greifschenkel (23, 24) im unbelasteten Zustand der Pinzette aneinander anliegen.

7. Chirurgische Einrichtung nach Anspruch 4 oder 5,
**dadurch gekennzeichnet, dass** die beiden Greifschenkel (23, 24) parallel zueinander verlaufen und so unter Spannung stehen, dass die beiden Kontaktflächen der Spitzen der beiden Greifschenkel (23, 24) im unbelasteten Zustand um ein vorbestimmtes Maß voneinander beabstandet sind und sich somit eine vorbestimmte Griffweite zwischen den Spitzen der beiden Greifschenkel (23, 24) einstellt.
